# EUROPEAN PATENT SPECIFICATION

(11) **EP 1 858 575 B1**
(45) Date of publication and mention of the grant of the patent: **26.05.2010**
(21) Application number: 06706103.6
(22) Date of filing: 03.03.2006
(51) Int. Cl.: A61M 25/00

(54) **A PACKAGE FOR A MEDICAL DEVICE**
VERPACKUNG FÜR EIN MEDIZINPRODUKT
EMBALLAGE DESTINE A UN DISPOSITIF MEDICAL

(30) Priority: 03.03.2005 DK 200500322; 03.03.2005 US 70283
(43) Date of publication of application: 28.11.2007
(73) Proprietor: Coloplast A/S, 3050 Humlebæk (DK)
(72) Inventor: BRUUN, Bo, Kjellman, DK-2100 Copenhagen Ø (DK); KLIXBÜLL, Lotte, M., DK-1721 Copenhagen V (DK); TORSTENSEN, Jan, DK-2830 Virum (DK)
(86) International application number: PCT/DK2006/000132
(87) International publication number: WO 2006/092150

(56) References cited:
- WO-A-99/30761
- DE-A1- 10 213 411
- US-A- 3 648 704
- US-A- 3 898 993
- US-A- 3 967 728
- US-A1- 2001 001 443
- US-A1- 2005 015 076

## Description

### INTRODUCTION

The invention relates to an assembly for wetting a medical device with a fluid medium, e.g. for wetting a catheter, such as a urinary catheter, e.g. with an antimicrobial agent, or a lubricant, or a saline solution for activating a hydrophilic low-friction surface. The assembly comprises a package accommodating the medical device and a compartment accommodating the fluid medium so that the fluid medium is not in contact with the medical device.

### BACKGROUND OF THE INVENTION

Often, medical devices such as catheters must be wetted with a liquid medium prior to use. As an example, it is typically desired to wet a medical device with an antimicrobial agent or with a substance for controlling the surface friction of the device. In one example, a medical catheter, e.g. a urinary catheter for draining the bladder, must be inserted into the body through a natural or artificial body passage, e.g. the urethra. To facilitate the insertion, a friction reducing substance is normally applied to the catheter. In the remaining part of this text, the invention is referred to in relation to a urinary catheter but the skilled person would readily derive other applications of the invention, e.g. catheters for blood vessels, respiratory system ventilation, etc.

Catheters for draining the bladder are used for intermittent as well as indwelling or permanent catheterisation. Typically, catheters are used by patients suffering from urinary retention, e.g. para- or tetraplegics who may have no control permitting voluntary urination. Catheters with low friction surface characteristics towards body tissue, e.g. a lubricated surface or a surface with a hydrophilic surface coating have been developed to facilitate insertion of the catheter into the body.

Typically, catheters are delivered in a completely sealed and sterilised package which, in addition to the catheter, may accommodate a substance which activates the low-friction characteristics of the catheter surface. Some of the existing packages provide the substance in a compartment which separates the substance from the catheter, e.g. in a pouch or in a small plastic bottle. Prior to the insertion of the catheter, the user must manipulate and empty the compartment for the content to be brought into contact with the catheter. Since the user's dexterity is sometimes reduced, the manipulation of the compartment inside the package can be difficult.

US 3,648,704 discloses a catheter apparatus with a cap member which is coupled to a casing member. The cap member is filled with a lubricating material. The cap member may be separated from the casing member by ripping the package along a line. The ripping action causes separation of the two members. Opening of the casing member and cap member requires tearing in a controlled direction being different from that of the prepared ripping line, and the catheter apparatus therefore provides no support for a combined opening action.

### DESCRIPTION OF THE INVENTION

It is an object of embodiments of the invention to facilitate wetting of a medical device, e.g. with respect to preparation of a urinary catheter before insertion into the body. Accordingly, the invention provides an assembly of the kind mentioned in the introduction and further comprising opening means adapted for a combined opening action whereby the package as well as the compartment are opened. The package comprises a container and a closure which can be detached from the container for making the medical device accessible and which interacts with the compartment to open the compartment upon movement of the closure relative to the container. The combination between the closure and container may in a simple and effective manner ensure that none of the enclosures, i.e. neither the package, nor the compartment, can be opened alone. This may prevent use of an untreated medical device and it may prevent treatment of a device without opening of the package. The invention may therefore provide a benefit e.g. when it is desired to avoid contact and thus treatment of the medical device until immediately prior to the use of the medical device. Due to the combined opening action, the compartment can be emptied as an integrated part of the opening procedure, and the risk of misuse, e.g. by forgetting to apply the fluid medium to the medical device prior to use, is reduced.

The combined opening action could be:
a) where the user opens the package and the compartment with one single grip in the assembly, e.g. by squeezing, compressing or bending the assembly,
b) where the user moves one single component relative to another component of the assembly, which movement thereby opens both the package and the compartment, either simultaneously or one by one. In one embodiment, a component of the assembly is moved back and/or forth In one single direction or rotated clockwise and/or anticlockwise whereby the compartment and the package open. The two components of the assembly which are moved relative to each other could e.g. be two portions of a package made from a flexible material, e.g. a foil material, or the two components could be two separate components, e.g. components which, at the delivery of the assembly to the user, are joined in a breakable adhesive joint. In another embodiment, relative movement between the catheter and the package may cause opening of the compartment.
   or
c) where establishing of access to the medical device by any intended opening method for the package automatically causes opening of the compartment. As an example, the package may comprise a cutting, tearing or rupturing feature, e.g. just an indication on the front surface, e.g. a line along which the user is intended to open the package, and the compartment may be located so that the tearing, cutting or rupturing also opens the compartment by the same opening action.

In this context, the word "opening of the package" means that the package is broken, ruptured, cut open, twisted apart, or in any way structurally prepared for making the medical device accessible.

That the package accommodates the medical device means that at least a portion of the medical device, typically a portion which is preferably maintained sterile until use, is protected inside a space formed by the package. The package could be of any suitable kind for the medical device in question, typically a package made from joined sheets of a foil material, or a hose or tube, e.g. made from a polymeric material.

That the fluid medium is not in contact with the medical device means that the fluid is incapable of interacting with the medical device until the compartment is opened.

Opening of the compartment means that the fluid medium becomes capable of being released from the compartment and thus becomes capable of interacting with the medical device.

The medical device could be of any kind, and as aforementioned, the device could be a catheter of the kind known in the art, i.e. comprising an elongate body extending between a proximal insertable tip and an axially opposite distal end, e.g. comprising a connector. The tip may form openings into an internal conduit for draining body fluids, e.g. urine, from the body through the catheter to a place of disposal. The connector could be provided e.g. for attaching a collection bag or for attaching a hose for an extension of the catheter. The catheter could also be of the kind forming axially extending outer grooves for conducting the urine along an outer surface.

The medical device could be surface coated, e.g. with a hydrophilic coating to be activated by a swelling medium, e.g. a saline solution.

The compartment could be a pouch, a bottle, a pocket forming part of the package, or any similar means for containing the fluid medium so that the medium is not in contact with the catheter. In one embodiment, one catheter is packed with several compartments which each contain a fluid medium which alone or in combination with the fluid medium of other packages provides an intended treatment of the medical device. One compartment may e.g. contain an antimicrobial agent and another compartment may contain a friction reducing substance.

In particular, the compartment may comprise an outlet, e.g. formed by a weak point at which the compartment easily ruptures, or formed by other means whereby the fluid medium can be emptied onto the catheter. The weak point could be constituted e.g. by a welding joint which is weak, or which contains a weak passage, or the weak point could be constituted by a reduced wall thickness of the compartment, or by a notch provided in an edge of the compartment to provoke rupturing upon application of a pressure thereto. In addition to, or as an alternative to the weak point, a cutting edge could be provided in an inner surface of the package to facilitate rupturing of the compartment upon contact with the cutting edge.

The fluid medium could be a liquid medium, a gas or powder. As an example, a liquid medium could be a saline solution or a similar medium for activating a low surface friction of a hydrophilic medical device, or the liquid could be a lubricant such as a hydrogel. The fluid medium may also comprise an active substance for treating a living being or the medium could comprise an antimicrobial agent. As an example, the medium could be an aqueous solution of an antimicrobial agent such as chlorhexidine digluconate, chlorhexidine dihydrochloride, benzalkonium chloride, hydrogen peroxide, silver chloride, silver sulfadiazine, silver hydantoinate, silver-5,5-dimethylhydantoinate or combinations thereof. In another example, the assembly contains a first substance e.g. in the form of a liquid, powder or gas which is contained in contact with the medical device, and the compartment contains another substance, e.g. a liquid, powder, or gas which - when the compartment is opened - reacts with the first substance to form an active substance which provides a desired functionality, e.g. renders the medical device low frictional or disinfected etc.

The assembly could be made so that the compartment and the package are opened essentially simultaneously, e.g. so that a seal of the package is broken at the time when the compartment is opened. The compartment could, however, also be opened prior to the opening of the package thereby allowing the fluid medium to wet the surface, or even to react with the surface before the package is opened, or the compartment could be opened after the package has been opened, e.g. as a consequence of removal or partly removal of the medical device from the package.

The assembly may have a shape which facilitates gripping, in particular for the user having a reduced dexterity. The opening means and possibly also other parts of the assembly may therefore be ergonomically shaped and made in a material, e.g. a synthetic material such as a soft rubber material, or with a surface texture, e.g. knobs, protrusions, ribs or depressions which improve handling, e.g. by the provision of a large surface friction or by the provision of a soft and deformable outer surface in which a handgrip can fixate the assembly or at least the opening means thereof. In one particular embodiment, the combined opening is facilitated by movement of a component relative to the reminder part of the assembly. The component may preferably protrude from the package to enable opening by pushing the component against an obstacle, e.g. a wall or a wash basin.

To establish contact between the fluid medium and the medical device, e.g. to wet a catheter with an antimicrobial or slippery liquid medium, the outlet may preferably be located adjacent the medical device, and preferably, the outlet may comprise a conduit which extends in a direction towards the medical device to establish a fluid flow from the compartment towards the device. If the medical device is a catheter to be inserted into the body of a living being, the outlet may advantageously be located close to, or possibly in direct contact with an insertable part of the catheter so that the fluid medium is applied directly to the part of the catheter where it is needed. In this way, contact between the fluid and parts of the catheter which are touched by the user, could be prevented.

In order further to prevent contact between the fluid and specific areas of the medical device, the assembly may further comprise isolating means, e.g. in the form of a gasket, a diaphragm etc. which is located in the package and which seals between inner walls of the package and outer walls of the catheter such that passage of the fluid between the surface of the catheter and the surface of the package is prevented. The gasket could be a ring shaped member located around the medical device. The gasket could be made from any suitable material, e.g. from a resilient material such as rubber or silicone. The gasket could even form part of the medical device or it could form part of the package, e.g. in the form of a protrusion of a surface of the device or package. If the medical device is a catheter, it may be an advantage to prevent fluid from entering into an inner conduit of the catheter. For that purpose, the opening inlets provided in the insertable proximal end of the catheter could be sealed, e.g. by a sealing structure which forms part of the package and which is thereby automatically removed from the catheter upon exposure of the insertable part of the catheter from the package, or upon complete removal of the catheter from the package.

The compartment could be held fixed at a location which, during normal handling of the assembly, is above the medical device. In that way, the gravity may be used for causing a flow of the fluid across the entire insertable surface of the device. To motivate arrangement of the assembly in a desired orientation to affect the above mentioned gravitationally aided spreading of the fluid, the assembly may contain a hanging structure, e.g. a hook, a hole, an adhesive strip or similar structure which facilitates hanging of the assembly in an orientation wherein the compartment is located above the medical device or at least above a portion of the medical device which is intended for contact with the fluid during the preparation of the device. Again, if the device is a catheter, the compartment could be located in the height of, or above the connector. During use, the connector part of the catheter is typically grabbed by the user for manipulating the catheter into, or out of the body, and a dry connector part facilitates this operation. To prevent the fluid from getting in contact with the connector, the package may comprise an elongate sleeve which narrowly encloses the insertable part of the catheter, and the outlet may be located in the sleeve for releasing the fluid directly onto the catheter at a position at a distance from the connector. In one embodiment, the sleeve may have a volume which is in the range of 1 to 20 times, e.g. in the range of 1 to 10 times, such as 1 to 5 times the volume of the insertable part of the catheter. The compartment should preferably contain a sufficient amount of the fluid medium to cause the intended effect on the medical device, e.g. to wet at least an insertable part of a catheter.

As aforementioned, a gasket could prevent the fluid from contaminating portions of the medical device which is not intended to be in contact with the fluid, e.g. fluid flowing from an insertable part of a catheter towards the connector part of the catheter. In this embodiment, a main portion of the compartment may be located on one side of the gasket and the outlet on the other side of the gasket so that the fluid can be stored at a position close to, e.g. directly adjacent the portion of the device which is not intended to be wetted, e.g. close to the connector part of a catheter while the fluid is released close to the portion which is intended for contact with the fluid, e.g. close to the insertable part of the catheter.

The package may comprise a container part and a detachable closure which interacts with the compartment to open the compartment upon movement of the closure relative to the container. The closure and the container could be joined in a threaded screw joint, by a releasable sealing strip, by an adhesive, by frictional resistance between the parts, or by any kind of engagement between the two parts. Analogously, the container part and the detachable closure could be separated prior to use by breaking, twisting, turning, rupturing squeezing or cutting the parts apart. Typically, the closed container and closure is delivered in a sterile condition.

In one embodiment, the closure forms part of, or is adhesively joined to the compartment in such a way that the compartment ruptures and opens upon removal of the closure from the container. In an alternative embodiment, the closure is located relative to the compartment to enable the closure to press against the compartment and thereby to rupture the compartment. To facilitate the rupturing of the compartment, a cutting edge could be located in the container or in the closure, or the edge could form part of the container or closure.

The closure may be designed so that release of the closure from the container requires opening of the compartment. The opening means may thus be adapted to open the compartment at the latest at the time when the package is opened. In one embodiment, the release of the closure may require the movement of the closure in a direction towards the compartment, and in another embodiment, the closure and compartment may be joined In such a way that the closure can be released, however not removed from the container without rupturing the compartment. In that way, the user may only be able to open, or at least only be able to gain access to the medical device in the intended way by also opening the compartment, and the user is therefore only able to remove the medical device during a procedure in which fluid medium is released onto the device.

If the medical device has an elongate shape, which is the case for most catheters, the compartment may encircle the catheter narrowly whereby a more homogenous wetting can be achieved and whereby space may be saved. As an example, the compartment may have an elongate shape which is either located lengthwise along the medical device or which is twisted around the medical device, or the compartment may comprise a through going hole through which the medical device can extend. The compartment could e.g. be ring-shaped.

The assembly may form storage space for one or more medical devices and/or for one or more compartments. In one embodiment, the assembly comprises a plurality of individually and mutually isolated packages for accommodation of a plurality of mutually isolated medical devices or a plurality of medical devices each having at least a portion which is isolated from the other medical devices. In this embodiment, one single compartment may be located with release means for releasing the liquid medium into one of, or all of the packages during a package opening action, or compartment may be located in connection with each package to wet the medical devices individually upon opening of the packages individually.

In a second aspect, the invention provides a method of wetting a medical device with a liquid medium contained in a compartment, said method comprising the step of placing the medical device and the compartment in a package so that the liquid medium and the medical device are not in direct contact. The method further comprising the step of opening the compartment and the package by the same opening action, e.g. in any of the aforementioned ways.

In a third aspect, the invention provides a compartment for an assembly according to the first aspect of the invention, which compartment is adapted to encircle the medical device in the package.

Any of the features described in connection with the first aspect may apply also to the second and third aspects.

### BRIEF DESCRIPTION OF THE DRAWINGS

Fig. 1 illustrates an opening sequence of an assembly according to the invention,
Fig. 2 illustrates an opening sequence of an alternative embodiment of the invention,
Fig. 3 illustrates an opening sequence of an alternative embodiment of the invention,
Fig. 4 illustrates a top view of an assembly according to the invention,
Figs. 5a and 5b illustrate different sealing joints between the container and top part of the assembly,
Fig. 6 illustrates an assembly with a gasket,
Fig. 7 illustrates an enlarged view of a top part of the assembly illustrated in Fig. 6,
Fig. 8 illustrates the use of the top part as an applicator for non contaminating manipulation of the catheter, and
Fig. 9 illustrates an alternative embodiment of the assembly.

### DETAILED DESCRIPTION OF THE PREFERRED EMBODIMENTS

Further scope of applicability of the present invention will become apparent from the following detailed description and specific examples.

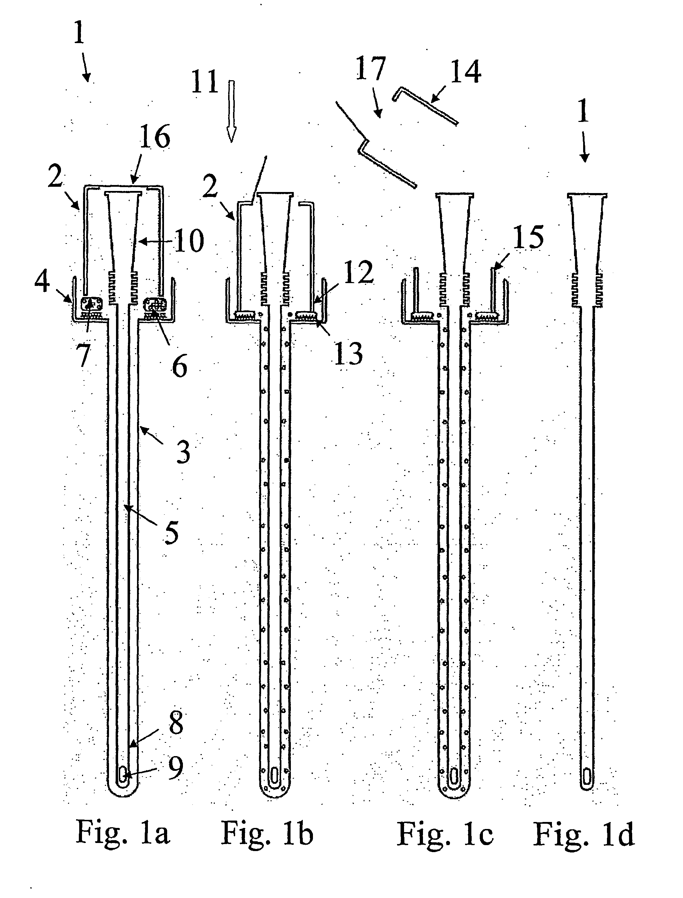

Fig. 1 illustrates an assembly for wetting a medical device with a liquid medium. The assembly comprises a package consisting of a closure 2 and a container comprising an elongate sleeve 3 which narrowly encloses the insertable part of the catheter and which is connected to a cup shaped top part 4 which is open upwardly. The package accommodates a urinary catheter 5 and further accommodates a compartment 6 with the liquid medium, indicated by bubbles 7. The liquid medium is kept separate from the catheter until the compartment is opened and emptied prior to use of the catheter. The catheter comprises a proximal end 8 shaped for insertion Into the body of the user and comprises inlet openings 9 for body substances to be drained into an inner conduit of the catheter. The opposite distal end of the catheter is provided with a connector part 10 from which the body fluids can be drained to a place of disposal. To reduce the amount of the liquid medium which is necessary for wetting the insertable part of the catheter, the container forms an elongate sleeve which narrowly encloses the proximal end of the catheter.

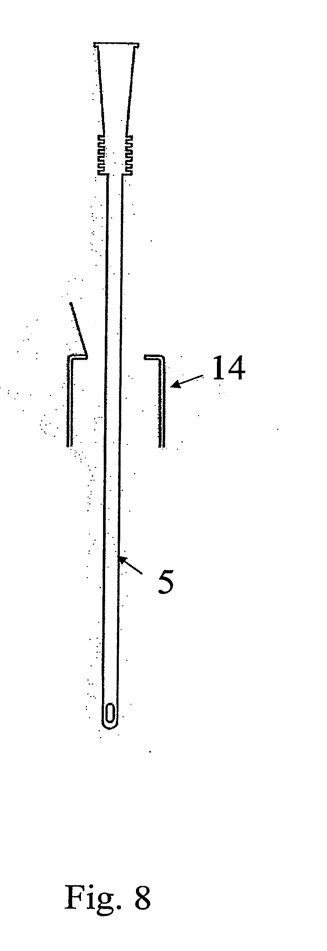

Fig. 1 illustrates the assembly 1 in four sequences of an opening procedure. In Fig. 1a, the catheter is sealed, e.g. hermetically, in the sterilised package. In Fig. 1b, the user has broken the seal between the closure 2 and the container 3, 4, by pushing the closure in the direction indicated by the arrow 11 whereby the edge 12 of the closure pushes the compartment towards the sharp pointed cutting edge 13. This breaks the sealing and the compartment is emptied whereby the liquid (indicated by the bubbles) flows downwardly into the elongate sleeve 3. In Fig. 1c, the closure is removed from the container to enable removal of the catheter from the package. To remove the closure, the user may either pull the closure in an upward direction, opposite the direction indicated by the arrow 11, or the user may break a top portion 14 of the closure free from a bottom portion 15. The disclosed assembly contains an additional opening feature consisting of a seal 16, e.g. a thin foil which is bonded to cover an opening 17 in a top face of the closure. During the initial pushing of the closure in the direction of the arrow it, the distal part of the catheter penetrates the foil or releases the foil from its contact with the closure to enable the catheter to be removed from the package without further opening of the package. The top portion 14 of the closure could be made from a soft, flexible material which could be squeezed into contact with the catheter merely by finger pressure, and the top portion may constitute an applicator facilitating non contaminating manipulation of the catheter without direct contact between the hands of the user and the catheter, c.f. also Fig. 8.

Due to the relationship between the distance between the catheter and the foil and the distance between the closure and the compartment, the compartment is opened prior to or, at the latest simultaneously with the opening of the package. In that way, removing the catheter from the package in the intended way before the compartment has been opened is prevented, and wetting of the catheter prior to use is ensured. In Fig. 1d, the catheter is removed from the package.

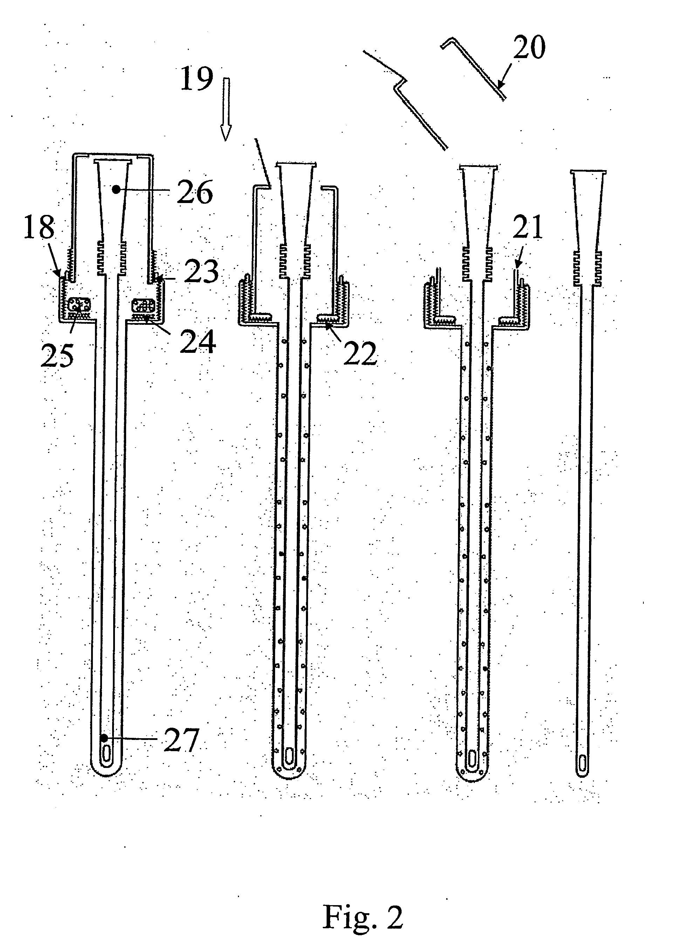

Fig. 2 illustrates an alternative embodiment of the assembly in which the closure is joined to the container via a threaded joint 18. To open the package, the user screws the closure as far as possible in the direction indicated by the arrow 19 until the top portion 20 of the closure breaks off from the bottom portion 21. At this point, the compartment has been pushed downwardly onto the sharp pointed edge 22 whereby it opens. The limitation of the travel of the closure in the downward direction could be defined e.g. by the length of the threaded inner surface of the container and/or by the threaded outer surface of the closure, or the travel may be defined by the edge 23 of the closure reaching the bottom 24 of the surface on which the compartment 25 is supported. Compared with the embodiment disclosed in Fig. 1, the compartment is located adjacent the catheter at a position more distant from the connector part 26 of the catheter, and the liquid substance is thus released closer to the proximal, insertable tip 27 on a part of the catheter which is to be inserted into the body of a patient and where a reduced friction and/or an improved antimicrobial protection are/is therefore particularly desired.

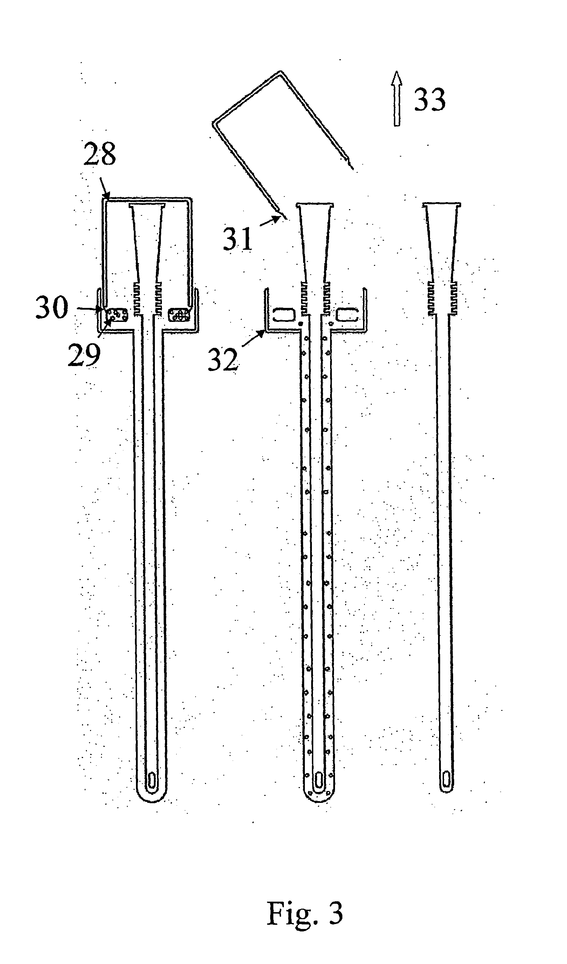

Fig. 3 illustrates an embodiment of the assembly in which the closure 28 forms part of, or is attached to the compartment 29 via the connecting portion 30 comprising a resilient strip 31 fastened to the closure and to the compartment. When the closure is removed from the container 32 in an upward direction, indicated by the arrow 33, the connecting portion follows the closure and thereby ruptures the compartment from which the content is discharged onto the insertable part of the surface of the catheter.

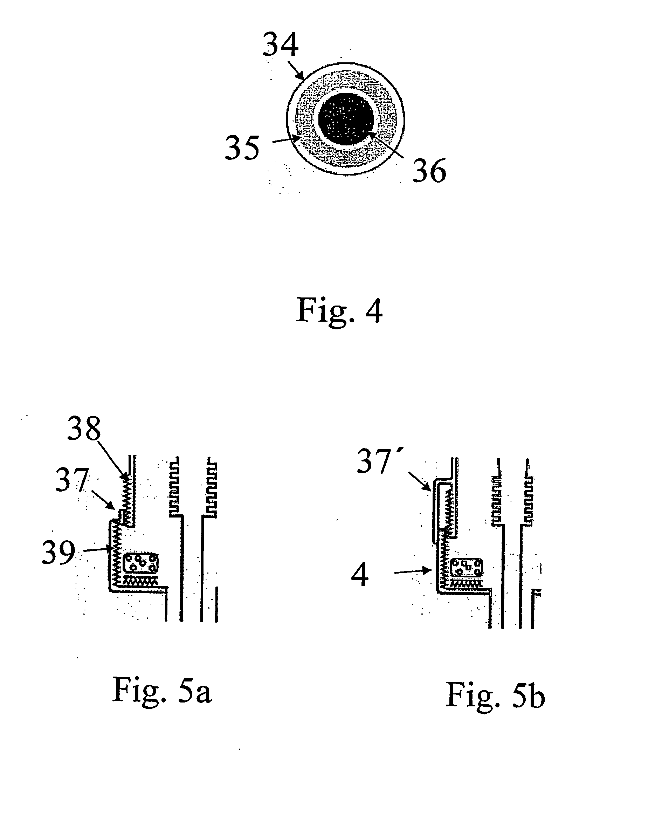

Fig. 4 illustrates a top view of the assembly in an embodiment with a tubular/circular shape. The outer periphery of the closure 34 encircles a compartment 35 which encircles the catheter 36.

Fig. 5a illustrates an assembly wherein a sealing is symbolized by sealing means 37 located between the threads 38 of the closure and the threads 39 of the container to seal the package. In Fig. 5b sealing means 37' is illustrated which is located to enclose the cup shaped top part 4.

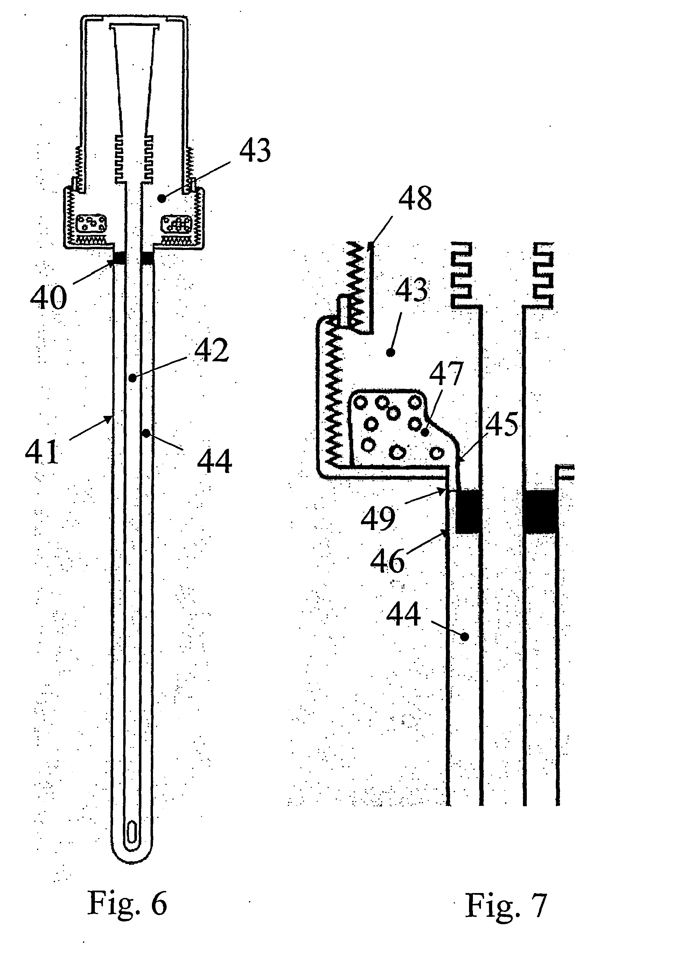

Fig. 6 illustrates an assembly wherein a gasket 40 is located between an inner surface of a sleeve-formed part 41 of the container and an outer surface of the catheter 42. The gasket separates the package into a first storage space 43 and a second storage space 44 between which the liquid is prevented from passing. The insertable part of the catheter is located in the second storage space and the connector is located in the first storage space.

Fig. 7 illustrates an enlarged view of one embodiment of an assembly with a gasket. The compartment comprises an elongate passage 45 which extends between the first and second spaces so that the compartment can be contained in the first space while the outlet 46 releases the liquid into the second space. The compartment 47 is made from a flexible material, and when the closure 48 is pushed downwardly towards the compartment, the internal pressure of the liquid increases to a point at which the seal 49 is severed and the liquid is emptied into the second space.

Fig. 8 Illustrates the use of the removable closure part or top portion 14 for noncontaminating insertion of the catheter. The top portion is made from a soft resilient material which after release from the container can be squeezed into engagement with the catheter 5 and be used to isolate the outer surface of the catheter from the hands of the user.

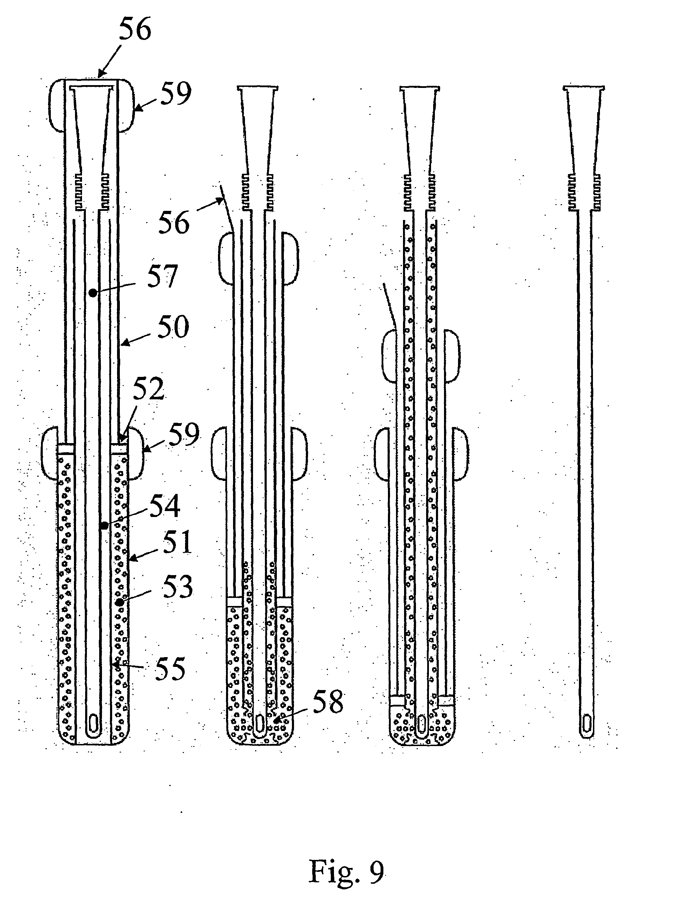

Fig. 9 illustrates an alternative embodiment of the invention wherein the container comprises a first section 50 and a second section 51 joined in a telescopic joint via the piston packing 52. The second section comprises a compartment 53 for the fluid medium and a cavity 54 for accommodation of the medical device. The fluid medium is separated from the medical device by the wall 55. The piston packing is attached to, or forms part of the first section and engages an inner surface of the second section. During the opening procedure, the first section is pushed into the second section, or more specifically, the first section is pushed into the compartment. During this movement of the first section relative to the second section, the top foil 56 is released whereby the catheter 57 or similar medical device is pushed out of the package, and the pressure of the fluid in the compartment is increased until a point where a section 58 of the wall 55 ruptures and the fluid medium, indicated by the bubbles, is pushed from the compartment into the cavity housing the medical device whereby the fluid and the device are brought in contact. To enable the rupturing of the wall 55, the wall may comprise a weak point, e.g. a notch or an incision or similar feature whereby the strength of the wall is reduced locally. As an alternative to the rupturing of the wall, the wall may comprise an opening which is sealed by a closure, e.g. a strip of a resilient tape etc. which is removed from the opening under influence of the increasing pressure of the fluid medium when the first section is moved relative to the second section. The protrusions 59 are provided to facilitate gripping of the package by the hands.

## Claims

1. An assembly (1) for wetting a medical device (5) with a fluid medium, said assembly comprising a package (2, 3, 4) accommodating the medical device, the assembly further comprising at least one compartment (6) accommodating the fluid medium so that the fluid medium is incapable of interacting with the medical device until the compartment is opened, **characterized in that** the package comprises a container (3, 4) and a closure (2) which can be detached from the container for making the medical device accessible and which interacts with the compartment to open the compartment upon movement of the closure relative to the container.

2. An assembly according to claim 1, wherein the opening means is adapted to open the package and the compartment by movement of a first component (2, 50) relative to a second component (3, 51) of the assembly.

3. An assembly according to claim 2, wherein one of the components (2, 3) compresses or damages the compartment (6) during movement of the first component (2) relative to the second component (3), whereby the compartment (6) is opened.

4. An assembly according to claims 2-3, wherein the first component (28) forms part of or adhesively joins the compartment (29) to rupture the compartment (29) upon movement of the first component (28) relative to the compartment (29).

5. An assembly according to claim 4, wherein the compartment (29) is fixed to a part of the package (32), whereby the compartment (29) ruptures upon movement of the first component (28) relative that part of the package (32).

6. An assembly according to claims 1-5, wherein the opening means is adapted to prevent exposure of the medical device without a preceding opening of the compartment.

7. An assembly according to any of the preceding claims, wherein the opening means is adapted for opening of the compartment at the latest simultaneously with opening of the package.

8. An assembly according to any of the preceding claims, wherein the closure interacts with the compartment to open the compartment upon removal of the closure from the package.

9. An assembly according to any of the preceding claims, comprising a cutting edge (13) which is formed to perforate the compartment upon operation of the opening means.

10. An assembly according to claim 9, wherein the cutting edge (13) forms part of the closure.

11. An assembly according to claims 9-10, wherein the cutting edge (13) forms part of the container.

12. An assembly according to any of the preceding claims, wherein the fluid medium contains an antimicrobial substance.

13. An assembly according to any of the preceding claims, wherein the package (2, 3, 4) accommodates the compartment (6).

14. An assembly according to any of the preceding claims, wherein the package accommodates a first fluid medium and the compartment accommodates a second fluid medium, wherein the first and second fluid media interacts to form an active substance for providing a desired treatment of the medical device prior to its use.

15. An assembly according to any of the preceding claims wherein the package forms an elongate sleeve (3) for accommodation of at least an insertable length of the medical device.

16. An assembly according to any of the preceding claims, wherein the medical device comprises an insertable part adapted to be inserted into the body of a living being and wherein the compartment (47) comprises an outlet (46) located to release the fluid medium onto the insertable part.

17. An assembly according to any of the preceding claims, wherein the medical device is a catheter.

18. An assembly according to any of the preceding claims, wherein the compartment (35) encircles the medical device (36).

19. A method of using the assembly of claim 1 for wetting a medical device with a fluid medium contained in at least one compartment said method comprsing the steps of placing the medical device and the compartment in a package according to claim 1 so that the fluid medium and the medical device are not in direct contact during storage, and opening the compartment and the package by movement of a closure relative to a container whereby the medical device is made accessible and the fluid medium is brought into contact with the medical device.

## Patentansprüche

1. Anordnung (1) zum Befeuchten einer medizinischen Vorrichtung (5) mit einem fluiden Medium, wobei die Anordnung aufweist:
eine Verpackung (2, 3, 4), in der die medizinische Vorrichtung untergebracht ist, sowie mindestens ein Compartment (6), in dem das fluide Medium so vorgesehen ist, dass das fluide Medium nicht in der Lage ist, mit der medizinischen Vorrichtung in Wechselwirkung zu treten, bis das Compartment geöffnet wird,
**dadurch gekennzeichnet, dass** die Verpackung einen Behälter (3, 4) und einen Verschluss (2) aufweist, der vom Behälter abgenommen werden kann, um die medizinische Vorrichtung zugänglich zu machen, und der mit dem Compartment so zusammenwirkt, dass das Compartment durch Bewegen des Verschlusses relativ zum Behälter geöffnet wird.

2. Anordnung nach Anspruch 1, bei der die Mittel zum Öffnen so ausgebildet sind, dass die Verpackung und das Compartment durch Bewegen eines ersten Bestandteils (2, 50) relativ zu einem zweiten Bestandteil (3, 51) der Anordnung geöffnet werden.

3. Anordnung nach Anspruch 2, bei der einer der Bestandteile (2, 3) das Compartment (6) während des Bewegens des ersten Bestandteils (2) relativ zum zweiten Bestandteil (3) zusammendrückt oder beschädigt, wodurch das Compartment (6) geöffnet wird.

4. Anordnung nach Anspruch 2 oder 3, bei welcher der erste Bestandteil (28) einen Teil des Compartments (29) bildet oder durch Klebung mit dem Compartment (29) verbunden ist, sodass das Compartment (29) beim Bewegen des ersten Bestandteils (28) relativ zum Compartment (29) aufgebrochen wird.

5. Anordnung nach Anspruch 4, bei der das Compartment (29) an einem Teil der Verpackung (32) befestigt ist, wodurch das Compartment (29) beim Bewegen des ersten Bestandteils (28) relativ zu diesem Teil der Verpackung (32) aufgebrochen wird.

6. Anordnung nach einem der Ansprüche 1-5, bei der die Mittel zum Öffnen so ausgebildet sind, dass eine Exposition der medizinischen Vorrichtung ohne ein vorheriges Öffnen des Compartments verhindert wird.

7. Anordnung nach einem oder mehreren der vorhergehenden Ansprüche, bei der die Mittel zum Öffnen so ausgebildet sind, dass das Compartment spätestens gleichzeitig mit dem Öffnen der Verpackung geöffnet wird.

8. Anordnung nach einem oder mehreren der vorhergehenden Ansprüche, bei welcher der Verschluss so mit dem Compartment in Wechselwirkung steht, dass das Compartment beim Entfernen des Verschlusses von der Verpackung geöffnet wird.

9. Anordnung nach einem oder mehreren der vorhergehenden Ansprüche, die eine Schneidkante (13) aufweist, die so ausgebildet ist, dass das Compartment beim Betätigen der Mittel zum Öffnen perforiert wird.

10. Anordnung nach Anspruch 9, bei der die Schneidkante (13) einen Teil des Verschlusses bildet.

11. Anordnung nach Anspruch 9 oder 10, bei der die Schneidkante (13) einen Teil des Behälters bildet.

12. Anordnung nach einem oder mehreren der vorhergehenden Ansprüche, bei der das fluide Medium eine antimikrobielle Substanz enthält.

13. Anordnung nach einem oder mehreren der vorhergehenden Ansprüche, bei der das Compartment (6) in der Verpackung (2, 3, 4) vorgesehen ist.

14. Anordnung nach einem oder mehreren der vorhergehenden Ansprüche, bei der ein erstes fluides Medium in der Verpackung untergebracht ist und ein zweites fluides Medium im Compartment untergebracht ist, wobei das erste und das zweite fluide Medium so miteinander wechselwirken, dass eine aktive Substanz gebildet wird, mit der eine gewünschte Behandlung der medizinischen Vorrichtung vor ihrer Verwendung erzielt wird.

15. Anordnung nach einem oder mehreren der vorhergehenden Ansprüche, bei der die Verpackung eine längliche Hülse (3) bildet, in der zumindest eine einführbare Länge der medizinischen Vorrichtung untergebracht ist.

16. Anordnung nach einem oder mehreren der vorhergehenden Ansprüche, bei der die medizinische Vorrichtung einen einführbaren Teil aufweist, der so ausgebildet ist, dass er in den Körper eines Lebewesens eingeführt werden kann, und bei der das Compartment (47) einen Ausgang (46) aufweist, der so angeordnet ist, dass das fluide Medium auf den einführbaren Teil freigesetzt wird.

17. Anordnung nach einem oder mehreren der vorhergehenden Ansprüche, bei der die medizinische Vorrichtung ein Katheter ist.

18. Anordnung nach einem oder mehreren der vorhergehenden Ansprüche, bei der das Compartment (35) die medizinische Vorrichtung (36) umgibt.

19. Verfahren der Verwendung der Anordnung nach Anspruch 1 zum Befeuchten einer medizinischen Vorrichtung mit einem fluiden Medium, das in mindestens einem Compartment enthalten ist, wobei das Verfahren folgende Schritte umfasst:
Anordnen der medizinischen Vorrichtung und des Compartments in einer Verpackung nach Anspruch 1 in der Weise, dass das fluide Medium und die medizinische Vorrichtung während der Lagerung nicht in direktem Kontakt miteinander stehen, und
Öffnen des Compartments und der Verpackung durch Bewegen eines Verschlusses relativ zu einem Behälter, wodurch die medizinische Vorrichtung zugänglich gemacht und das fluide Medium mit der medizinischen Vorrichtung in Kontakt gebracht wird.

## Revendications

1. Ensemble (1) destiné à humidifier un dispositif médical (5) avec un milieu fluide, ledit ensemble comprenant un emballage (2, 3, 4) abritant le dispositif médical, l'ensemble comprenant en outre au moins un compartiment (6) abritant le milieu fluide de telle sorte que le milieu fluide soit incapable d'interagir avec le dispositif médical avant que le compartiment ne soit ouvert, **caractérisé en ce que** l'emballage comprend un récipient (3, 4) et une fermeture (2) qui peut être détachée du récipient afin de rendre le dispositif médical accessible et qui interagit avec le compartiment pour ouvrir le compartiment lors du mouvement de la fermeture par rapport au récipient.

2. Ensemble selon la revendication 1, dans lequel le moyen d'ouverture est adapté à ouvrir l'emballage et le compartiment par mouvement d'un premier composant (2, 50) par rapport à un deuxième composant (3, 51) de l'ensemble.

3. Ensemble selon la revendication 2, dans lequel l'un des composants (2, 3) comprime ou détériore le compartiment (6) au cours du mouvement du premier composant (2) par rapport au deuxième composant (3), moyennant quoi le compartiment (6) est ouvert.

4. Ensemble selon les revendications 2 à 3, dans lequel le premier composant (28) fait partie ou est joint par adhésion au compartiment (29) afin de rompre le compartiment (29) lors du mouvement du premier composant (28) par rapport au compartiment (29).

5. Ensemble selon la revendication 4, dans lequel le compartiment (29) est fixé à une partie de l'emballage (32), moyennant quoi le compartiment (29) se rompt lors du mouvement du premier composant (28) par rapport à cette partie de l'emballage (32).

6. Ensemble selon les revendications 1 à 5, dans lequel le moyen d'ouverture est adapté à éviter une mise à nu du dispositif médical sans ouverture préalable du compartiment.

7. Ensemble selon l'une quelconque des revendications précédentes, dans lequel le moyen d'ouverture est adapté à l'ouverture du compartiment au plus tard simultanément à l'ouverture de l'emballage.

8. Ensemble selon l'une quelconque des revendications précédentes, dans lequel la fermeture interagit avec le compartiment pour ouvrir le compartiment lors du retrait de la fermeture de l'emballage.

9. Ensemble selon l'une quelconque des revendications précédentes, comprenant une arête tranchante (13) qui est formée dans le but de perforer le compartiment lors de l'actionnement du moyen d'ouverture.

10. Ensemble selon la revendication 9, dans lequel l'arête tranchante (13) fait partie de la fermeture.

11. Ensemble selon les revendications 9 à 10, dans lequel l'arête tranchante (13) fait partie du récipient.

12. Ensemble selon l'une quelconque des revendications précédentes, dans lequel le milieu fluide comprend une substance antimicrobienne.

13. Ensemble selon l'une quelconque des revendications précédentes, dans lequel l'emballage (2, 3, 4) abrite le compartiment (6).

14. Ensemble selon l'une quelconque des revendications précédentes, dans lequel l'emballage abrite un premier milieu fluide et le compartiment abrite un deuxième milieu fluide, dans lequel les premier et deuxième milieux fluides interagissent pour former une substance active permettant de réaliser un traitement désiré du dispositif médical avant son utilisation.

15. Ensemble selon l'une quelconque des revendications précédentes, dans lequel l'emballage forme un manchon allongé (3) destiné à abriter au moins une longueur insérable du dispositif médical.

16. Ensemble selon l'une quelconque des revendications précédentes, dans lequel le dispositif médical comprend une partie insérable adaptée à être insérée dans le corps d'un être vivant et dans lequel le compartiment (47) comprend un orifice de sortie (46) aménagé dans le but de libérer le milieu fluide sur la partie insérable.

17. Ensemble selon l'une quelconque des revendications précédentes, dans lequel le dispositif médical est un cathéter.

18. Ensemble selon l'une quelconque des revendications précédentes, dans lequel le compartiment (35) entoure le dispositif médical (36).

19. Procédé d'utilisation de l'ensemble selon la revendication 1 dans le but d'humidifier un dispositif médical avec un milieu fluide contenu dans au moins un compartiment, ledit procédé comprenant les étapes consistant à placer le dispositif médical et le compartiment dans un emballage selon la revendication 1, de telle sorte que le milieu fluide et le dispositif médical ne soient pas en contact direct au cours du stockage, et à ouvrir le compartiment et l'emballage grâce au mouvement d'une fermeture par rapport à un récipient, moyennant quoi le dispositif médical est rendu accessible et le milieu fluide est mis en contact avec le dispositif médical.
